# EUROPEAN PATENT APPLICATION

(11) **EP 1 491 218 A1**
(43) Date of publication of application: **29.12.2004**
(21) Application number: 03012452.3
(22) Date of filing: 30.05.2003
(51) Int. Cl.: A61L 9/18, A61L 9/20, B01D 53/86, B01D 53/88, B01J 19/12, B01J 35/00, A62D 3/00, F24F 3/16, H01J 61/02, H01J 61/35

(54) **Illuminator capable of cleaning air**

(71) Applicant: Taiwan Fluorescent Lamp Co., Ltd., Taipei City, Taiwan (TW)
(72) Inventor: Huang, Chu-Chun, Chu Tung Jen, Hsin Chu Hsien (TW); Cheng, Wen-Hu, Hsin Chu Hsien (TW)
(74) Representative: Viering, Jentschura & Partner

(57) **Abstract**

An illuminator capable of cleaning air is disclosed. The illuminator includes a light emitting body (40), a fiberglass carrier (20) covering the light emitting body, a photocatalytic film (10) mounted on the fiberglass carrier having therein a plurality of irregular paths (11), and a mounting piece (30) for fixing the fiberglass carrier (20) on the light emitting body (40).

## Description

### BACKGROUND OF THE INVENTION

### 1. Field of the Invention

The present invention relates to an illuminator capable of cleaning air.

### 2. Description of the Related Art

In recent years, there has been a growing interest in globally environmental pollution. Meanwhile, the demand for removing substances has grown. Moreover, a plan for creating amenity space by eliminating toxic substances has been devised. Thus, the demands for deodorizing living space and for making the living space antibacterial, soil-resistant and mildew-proof have grown increasingly. Photocatalyst such as titanium dioxide (TiO₂), which decomposes organic matter using energy of ultraviolet radiation, is known in the art. The capability of producing powerful oxidizing hydroxyl radicals enables titanium dioxide to be anti-bacterial, non-toxic, deodorizing, hydrophilic, and self-cleaning. These properties make titanium dioxide an ideal environmental protection product.

Generally speaking, photocatalyst is a substance that, after illuminated by light, can enhance a chemical reaction although the substance itself will not undergo changes. Photocatalyst can be divided into organo-metallic compounds (pigment) and semiconductor. After illuminated by light, semiconductor photocatalyst can control a chemical reaction. It can perform functions such as disinfection, anti-bacterial, bactericidal, anti-pollution and removal of harmful substances etc. As a result, extensive research has been carried out to explore and make use of the properties of photocatalyst in our everyday life.

When titanium dioxide absorbs Ultraviolet (UV) radiation from sunlight or illuminated light sources, it will produce electron-hole pairs, which are mobile charge carriers carrying negative and positive charge respectively. Specially treated titanium dioxide can be sprayed on window to form a thin film that is antifogging and heat tolerable up to 400 degree Celsius. It is also water stainless due to its hydrophilic nature. When dust particles fall on a titanium dioxide treated surface, it can be removed by clean water. The hydrophilic nature of titanium dioxide, coupled with the gravity, will enable the dust particles to be swept away following the water stream, thus making the product self-cleaning.

U.S. Pat. No. 5,439,652, filed September 30, 1993 by Sczechowski et al., discloses a photocatalytic method for removing organic contaminants from fluid or gas phases and a photoreactor design which allows exposure of photoreactive material to controlled periodic illumination.

U.S. Pat. No. 5,501,801, filed November 30, 1993 by Zhang et al., assigned to Board of Control of Michigan Technology University (Houghton, MI) discloses an apparatus for the photocatalytic oxidation of organic contaminants in fluid includes a reactor and a photocatalyst affixed to support material. The outer wall of the reactor is constructed of material transmissive of ultraviolet radiation. The support material preferably is transmissive of ultraviolet radiation. The support material can also be an adsorbent material.

U.S. Pat. No. 5,480,524, filed May 3, 1994 by Oeste, discloses a method and pertinent apparatus for purifying gases, exhaust gases, vapors, and brines, which are contaminated with undesirable chemical substances or contain high concentrations of these substances, by means of photocatalytic reactions occurring on the surface of catalysts. The catalysts are situated in a fixed or fluidized bed on catalyst carriers. In fluidized beds, the catalysts themselves can serve as catalyst carriers. The substrates to be purified are fed through a closed system which contains the catalyst carriers and catalysts. In the fixed-bed catalytic method, the catalyst carrier/catalyst system continuously or discontinuously passes through a washing zone to remove the generated mineralization products. The reaction is induced by shortwave photons of wavelengths between 250 and 400 nm.

### SUMMARY OF THE INVENTION

One object of the invention is to provide an illuminator capable of cleaning air for decomposing airborne organic substances, eliminating odors, killing bacterium in the air.

It is another object of the present invention is to provide an illuminator capable of cleaning air with self-cleaning function and air convection.

Still another of the present invention is to provide an illuminator capable of cleaning air and providing soft lighting.

Still another of the present invention is to provide an illuminator capable of cleaning air having a porous photocatalytic film that provides irregular paths to increase total surface of the porous photocatalytic film.

Still another of the present invention is to provide an illuminator capable of cleaning air having a simple structure and is thus cost-effective.

To achieve these and other advantages and in accordance with the purposes of the present invention, as embodied and broadly described herein, the present invention provides an illuminator capable of cleaning air comprising a light emitting body; a fiberglass carrier covering the light emitting body; a photocatalytic film mounted on the fiberglass carrier having therein a plurality of irregular paths; and a mounting piece for fixing the fiberglass carrier on the light emitting body.

Other objects, advantages and novel features of the invention will become more clearly and readily apparent from the following detailed description when taken in conjunction with the accompanying drawings.

### BRIEF DESCRIPTION OF THE DRAWINGS

- Fig.1: is a schematic perspective view depicting a fiberglass carrier coated with a photocatalytic film in accordance with the present invention.
- Fig.2: is a schematic cross-sectional view of the fiberglass carrier coated with a photocatalytic film emphasizing the irregular paths therein in accordance with the present invention.
- Fig.3: is a perspective view showing the lamp device wound by fiberglass carrier coated with a photocatalytic film in accordance with the present invention.
- Fig.4: is a schematic view of woven fiberglass in accordance with the present invention.
- Fig.5: is a schematic cross-sectional diagram showing the structure of the photocatalytic film doped with oxidizing catalyst particles in accordance with the present invention.
- Fig.6: is a schematic cross-sectional diagram showing the lamp device emphasizing the mounting piece in accordance with the present invention.
- Fig.7: is a perspective view showing a UV lamp wound by multi-layer fiberglass in accordance with the present invention.
- Fig.8: is a perspective view showing a UV lamp covered by a bag shape fiberglass in accordance with the present invention.
- Fig.9: is a perspective view showing an annular UV lamp covered by fiberglass in accordance with the present invention.
- Fig. 10 to Fig. 12: are plots presenting the butyl-acetate gas removal rate.

### DETAILED DESCRIPTION OF THE PREFERRED EMBODIMENTS

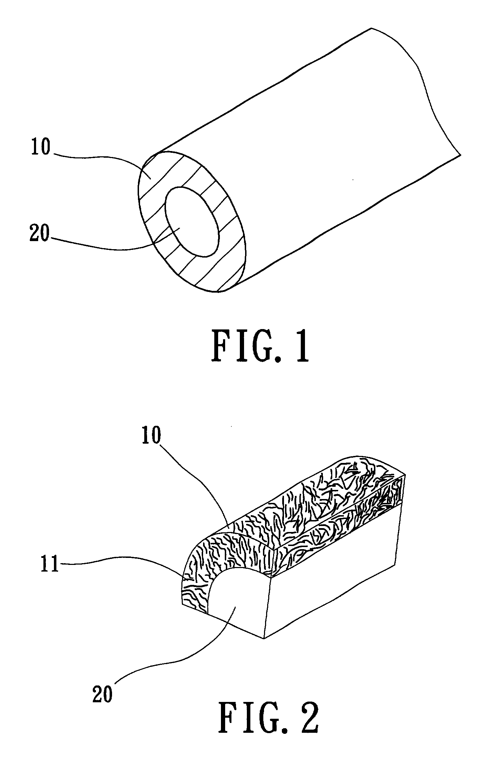

Referring to Fig.1 to Fig.3, the present invention pertains to an illuminating device capable of purifying air. A photocatalytic film 10 is coated on a fiberglass carrier 20 by using a sol-gel method, followed by a sintering process. Irregular paths 11 are formed in the photocatalytic film 10. A mounting piece 30 is used to fix the fiberglass carrier 20 onto an outer surface of a light emitting body 40 that emits 250nm - 760nm photons to irradiate the photocatalytic film 10 and thus generating electron-hole pairs, which are mobile charge carriers carrying negative and positive charge respectively. The hole has strong oxidizing power and the electron has strong reducing power. When these electron-hole pairs react with moisture on the surface of the titanium dioxide, powerful oxidizing hydroxyl radicals like O, O₂, OH etc. will be produced. These hydroxyl radicals can provide different treatments to treat bacteria (organic matters) and odor (organic gases) specifically.

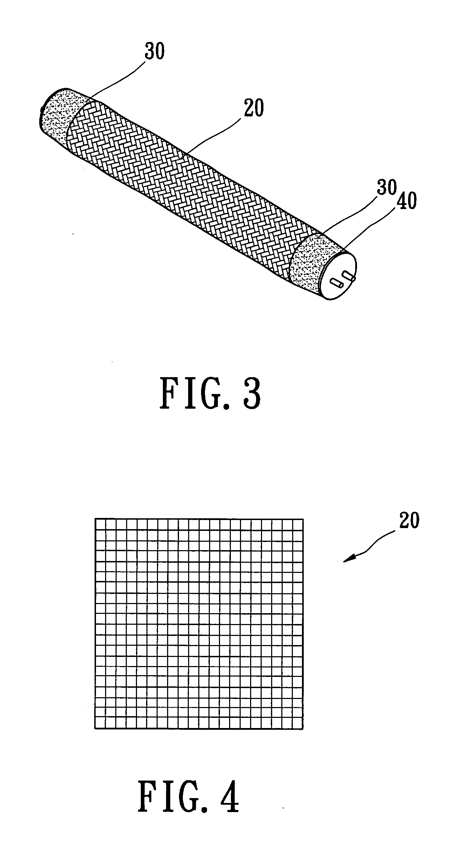

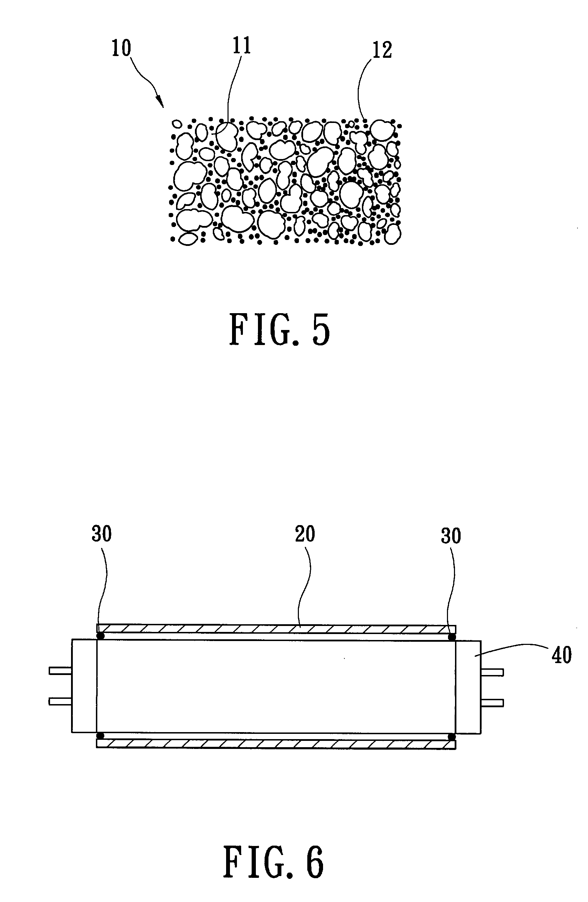

The fiberglass carrier 20 may be in the form of gauze or woven fiberglass, as shown in Fig.4. In either case, the diameter of fiberglass is between 10 micrometers to 100 micrometers to form 1 to 100-count fiberglass gauze with a plurality of tiny apertures therein. These tiny apertures increase the total surface for accommodating the photocatalytic film 10. Oxidizing catalyst particles are distributed on the irregular paths 11, as shown in Fig.5. Organic substances in the air diffuse to the active sites of the photocatalytic film 10 and are destroyed or decomposed.

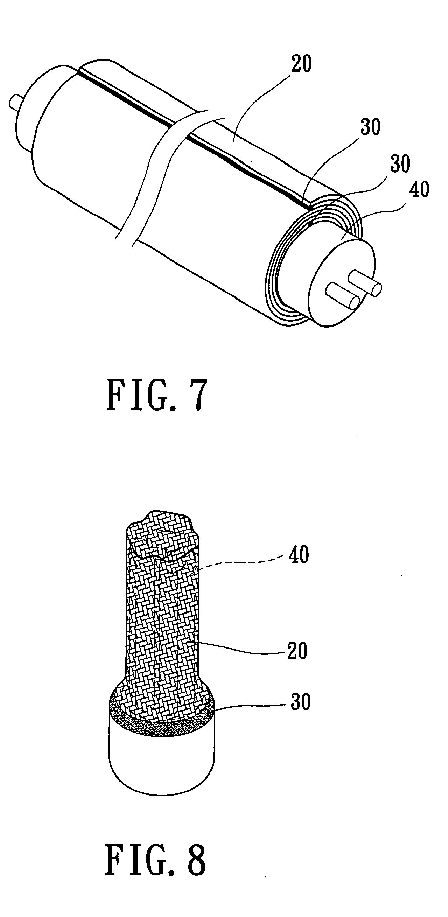

The fiberglass carrier 20 may be cylindrical woven fiberglass, as shown in Fig.3 and Fig.6, so that it can be a sleeve installed on a light emitting body 40. Or, the fiberglass carrier 20 may be in a strap form to wind the light emitting body 40, as shown in Fig.7, so that the light emitting body 40 can be wound by multi-layer fiberglass carrier 20. Alternatively, the fiberglass carrier 20 may be in a bag form, as shown in Fig.8, so that the light emitting body 40 can be covered by the fiberglass bag. The mounting piece 30, which is used to fix the fiberglass carrier 20 on the light emitting body 40, may be UV-resistant glue such as silicone or glass glue, as shown in Fig.6 and Fig.7, or a fasten ring, as shown in Fig. 3 and Fig.8. Preferably, the fasten ring is made of thermoplastic resins or other equivalent flexible materials.

Referring to Fig.3 and Fig.6, the light emitting body 40 may be an ultraviolet (UV) lamp capable of emitting 254nm or 365nm UV light. The 254nm UV lamp may serve as an anti-bacteria lamp. When using the 254nm UV lamp as a UV light source, the woven fiberglass covering the lamp body has a lower weaving density. The sparse woven fiberglass allows UV light penetrating therethrough to kill bacteria. To achieve the same goal of passing UV light, only a portion of the UV lamp surface is covered by the woven fiberglass. A portion of UV light is absorbed by the photocatalytic film 10 to generate active radicals to kill bacteria or to decompose unwanted substances. When using the 365nm UV lamp as a UV light source, the woven fiberglass covering the lamp body has a higher weaving density to ensure that the UV light is completely absorbed by the photocatalytic film 10. In this case, the fiber counts and weaving density of woven fiberglass is decided depending on experiences. Ideally, air may flow through the paths 11 of the photocatalytic film 10 in a convective manner without leaking the UV light out. In another case, multi-layer woven fiberglass is wound on the UV lamp body to prevent the UV light from leaking out..

Referring to Fig.2 and Fig.3, the UV lamp body may be made of sodium calcium glass, quartz, boron silicate glass, or equivalent materials that will not block the UV light. To ensure that sufficient amount of UV light can be absorbed by the photocatalytic film 10, the sodium calcium glass is the preferred candidate due to its highly transparence to UV light. To gain higher surface area of the photocatalytic film 10, the sol-gel method is used to coat the photocatalytic film 10 on the fiberglass carrier 20.

The sol-gel method involves the use of alcoholic metal salt Ti(OR)4 as a main material. Certain organic and inorganic slats are added into the alcoholic solution to carry on hydration reaction, thereby obtaining organic metal compounds in gel form. The gel type organic metal compounds is blended with other organic or inorganic photocatalyst such as WO₃, SnO₂, Fe₂O₃, or ZnO. After preparing the gel solution as described above, the fiberglass carrier 20 is dipped in the gel solution. By controlling the extracting speed of the fiberglass carrier 20 from the gel solution, one can obtain a desired thickness of the photocatalytic film 10. The fiberglass carrier 20 coated with photocatalytic film 10 is then stored for a period of time. Then, the fiberglass carrier 20 is subjected to baking, following by sintering at high temperatures. To improve performance of the illuminating device capable of purifying air of this invention, oxidizing catalyst particles such as noble metals or transition metals are added. Preferably, some noble metals such as Au, Pd, Pt, or Ag, and some transistion metals such as MoO₃, Nb₂O₅, V₂O₅, CeO₂, or Cr₂O₃ may be added to promote decomposition efficiency of the photocatalytic reaction.

Referring to Fig.1, Fig.2, Fig.4 and Fig.5, the method of adding the oxidizing catalyst particles includes the steps of dipping the fiberglass carrier 20 coated with photocatalytic film 10 into solution containing oxidizing catalyst salts. Since the fiberglass carrier 20 is a porous structure and thus contains a plurality of paths 11, these oxidizing catalyst salts will enter the paths 11. The fiberglass carrier 20 coated with photocatalytic film 10 blended with oxidizing catalyst is then subjected to baking and activation.

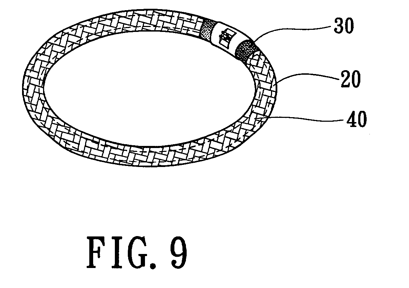

The lamp body may have different shapes such as annular-shape (as shown in Fig.9), U-shape, ball-shape, pillar-shape, or spiral-shape. Heat generated by the lamp body facilitates the convection and thus increasing the anti-bacteria efficiency. It is one of the main features of the present invention that the photocatalytic film 10 can absorb UV light emitted by the lamp device and decompose the adsorbed substances. It is another feature of the present invention that the photocatalytic film 10 can be used to diffuse visible light for providing a more comfortable lighting.

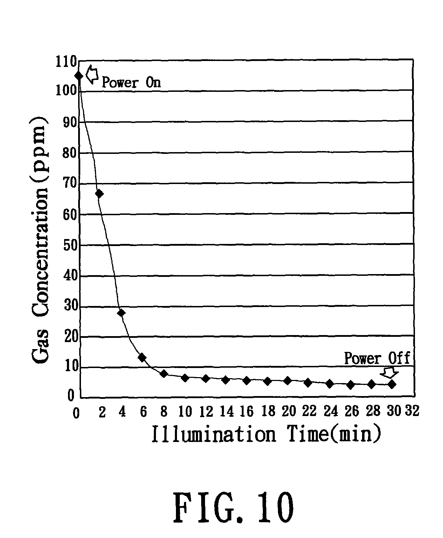

Referring to Fig.10 to Fig. 12, experimental gas removal efficiency is presented. The experimental data are obtained by using a gas detector that is installed in a hermetically sealed vessel, in which an amount of (4L) gaseous butyl-acetate is injected in advance. The illuminator device of this invention is installed in the vessel. As shown in Fig.10, after 4-minute irradiation of 254nm light by the light emitting body 40, the concentration of butyl-acetate is below 30ppm. After 6-minute irradiation of 254nm light by the light emitting body 40, the concentration of butyl-acetate is about 10ppm. After 8-minute irradiation of 254nm light by the light emitting body 40, the concentration of butyl-acetate is below 10ppm.

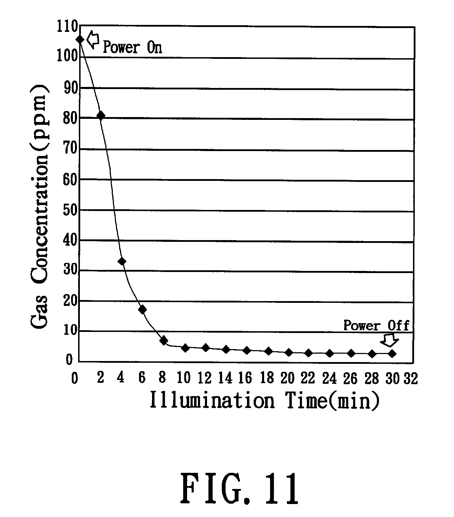

As shown in Fig.11, after 4-minute irradiation of 365nm light by the light emitting body 40, the concentration of butyl-acetate is about 30ppm. After 6-minute irradiation of 365nm light by the light emitting body 40, the concentration of butyl-acetate is below 20ppm. After 8-minute irradiation of 365nm light by the light emitting body 40, the concentration of butyl-acetate is below 10ppm.

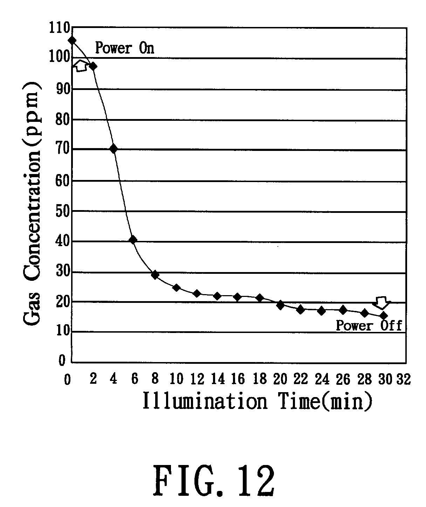

As shown in Fig.12, after 4-minute irradiation of 543nm light by the light emitting body 40, the concentration of butyl-acetate is about 70ppm. After 6-minute irradiation of 543nm light by the light emitting body 40, the concentration of butyl-acetate is about 40ppm. After 8-minute irradiation of 543nm light by the light emitting body 40, the concentration of butyl-acetate is below 30ppm. From above, using the 543nm light source results in highest remaining butyl-acetate concentration at 4-minute level, while using the 254nm light source results in lowest remaining butyl-acetate concentration. At a 6-minute level, using the 543nm light source results in highest remaining butyl-acetate concentration at 4-minute level, while using the 254nm light source results in lowest remaining butyl-acetate concentration. At the 8-minute level, both using the 254nm and 365nm slight sources result in a remaining butyl-acetate concentration that is below 10ppm.

To sum up, the present invention provides an illuminating device capable of purifying air. The illuminator according to this invention not only can provide soft lighting (light can diffused or scattered by the photocatalytic film 10), but also can efficiently decompose airborne organic substances. The illuminator of this invention can be used to eliminate unpleasing odor in a room or in an office and also kill bacterium because it has larger contact area provided by the fiberglass. Further, the devcie has a simple structure and is more cost-effective.

It is to be understood, however, that even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. An illuminator capable of cleaning air, comprising:
a light emitting body;
a fiberglass carrier covering the light emitting body;
a photocatalytic film mounted on the fiberglass carrier having therein a plurality of irregular paths; and
a mounting piece for fixing the fiberglass carrier on the light emitting body.

2. The illuminator capable of cleaning air as claimed in claim 1 wherein the fiberglass carrier is woven fiberglass.

3. The illuminator capable of cleaning air as claimed in claim 1 wherein the fiberglass carrier is porous fiberglass gauze.

4. The illuminator capable of cleaning air as claimed in claim 1 wherein the fiberglass carrier is shaped into cylindrical woven fiberglass.

5. The illuminator capable of cleaning air as claimed in claim 1 wherein the light emitting body is wound by the fiberglass carrier.

6. The illuminator capable of cleaning air as claimed in claim 1 wherein the fiberglass carrier is shaped into a bag type woven fiberglass and the light emitting body is covered by the bag type woven fiberglass.

7. The illuminator capable of cleaning air as claimed in claim 1 wherein the light emitting body emits 365nm light.

8. The illuminator capable of cleaning air as claimed in claim 1 wherein the light emitting body emits 312nm light.

9. The illuminator capable of cleaning air as claimed in claim 1 wherein the light emitting body emits 254nm light.

10. The illuminator capable of cleaning air as claimed in claim 1 wherein the photocatalytic film comprises TiO₂, WO₃, SnO₂, Fe₂O₃, or ZnO.

11. The illuminator capable of cleaning air as claimed in claim 1 further comprising oxidizing catalyst particles distributed and fixed on the paths of the photocatalytic film.

12. The illuminator capable of cleaning air as claimed in claim 11 wherein the oxidizing catalyst particles include noble metals.

13. The illuminator capable of cleaning air as claimed in claim 12 wherein the noble metal comprises Au, Pd, Pt, or Ag.

14. The illuminator capable of cleaning air as claimed in claim 11 wherein the oxidizing catalyst particles include transition metal oxides.

15. The illuminator capable of cleaning air as claimed in claim 14 wherein the transition metal oxide comprises MoO₃, Nb₂O₅, V₂O₅, CeO₂, or Cr₂O₃.

16. The illuminator capable of cleaning air as claimed in claim 1 wherein the mounting piece is an annular ring.

17. The illuminator capable of cleaning air as claimed in claim 16 wherein the annular ring is made of thermoplastic resins.

18. The illuminator capable of cleaning air as claimed in claim 16 wherein the annular ring is made of flexible materials.

19. The illuminator capable of cleaning air as claimed in claim 1 wherein the mounting piece is glue that is adhered to an outer surface of the light emitting body.

20. The illuminator capable of cleaning air as claimed in claim 19 wherein the glue is silicone.

21. The illuminator capable of cleaning air as claimed in claim 19 wherein the glue is glass glue.

22. The illuminator capable of cleaning air as claimed in claim 1 wherein the light emitting body is ball-shaped.

23. The illuminator capable of cleaning air as claimed in claim 1 wherein the light emitting body is pillar-shaped.

24. The illuminator capable of cleaning air as claimed in claim 1 wherein the light emitting body is U-shaped.

25. The illuminator capable of cleaning air as claimed in claim 1 wherein the light emitting body is spiral-shaped.

26. The illuminator capable of cleaning air as claimed in claim 1 wherein the light emitting body emits light having a wavelength between 380nm to 760nm.
